**Europäisches Patentamt**

**European Patent Office**

**Office européen des brevets**

(11) Veröffentlichungsnummer: **0 218 006**
**B1**

(12) **EUROPÄISCHE PATENTSCHRIFT**

(45) Veröffentlichungstag der Patentschrift:
**29.11.89**

(21) Anmeldenummer: **86107945.7**

(22) Anmeldetag: **11.06.86**

(51) Int. Cl.⁴: **A61N 1/36**

(54) **Vorhofgesteuerter Herzschrittmacher.**

(30) Priorität: **04.10.85 DE 3535517**

(43) Veröffentlichungstag der Anmeldung:
**15.04.87 Patentblatt 87/16**

(45) Bekanntmachung des Hinweises auf die Patenterteilung:
**29.11.89 Patentblatt 89/48**

(84) Benannte Vertragsstaaten:
**DE FR GB IT NL SE**

(56) Entgegenhaltungen:
**FR-A- 2 516 797**
**US-A- 3 939 844**

(73) Patentinhaber: **Siemens Aktiengesellschaft,
Wittelsbacherplatz 2, D-8000 München 2(DE)**

(84) Benannte Vertragsstaaten: **DE FR GB IT NL**

(73) Patentinhaber: **Siemens Elema AB, Röntgenvägen 2,
S-171 95 Solna 1(SE)**

(84) Benannte Vertragsstaaten: **SE**

(72) Erfinder: **Hedberg, Sven-Erik, Dipl.-Ing., Odonstigen 5,
S-196 31 Kungsängen(SE)**
Erfinder: **Lindgren, Anders, Dipl.-Ing., Odonstigen 5,
S-196 31 Kungsängen(SE)**

## Beschreibung

Ein vorhofgesteuerter Herzschrittmacher weist einen mit den P-Wellen synchronen Impulsgenerator auf, der zwischen einer programmierten Basisfrequenz und einer Höchstfrequenz kontinuierlich arbeitet. Bei einem Herzschrittmacher dieser Art liegt ein geschlossener Kreis vor, der vom Herzschrittmacherausgang über die Stimulationselektrode, die Herzkammer, die Herzwand und den Vorhof zum Herzschrittmacher verläuft. Dies bedeutet, daß die Stimulationsimpulse über das Herz auf den Herzschrittmacher rückgekoppelt werden können. Dadurch kann eine Tachykardie auftreten.

Der Erfindung liegt die Aufgabe zugrunde, einen Herzschrittmacher der eingangs genannten Art so auszubilden, daß eine durch den Herzschrittmacher erzeugte Tachykardie automatisch beendet wird.

Diese Aufgabe ist erfindungsgemäß gelöst durch eine Überwachungsschaltung für die Frequenz der erzeugten P-Wellen, die die Weiterleitung einer P-Welle unterbricht, wenn diese Frequenz einen vorbestimmten Wert überschreitet. Bei dem erfindungsgemäßen Herzschrittmacher kann durch einen Fenstergenerator ein Fenster vorgegeben werden, innerhalb dessen eine vorbestimmte Höchstzahl n von P-Wellen auftreten darf. Wenn innerhalb des Fensters die n-te P-Welle auftritt, ist dies ein Zeichen dafür, daß eine durch den Herzschrittmacher erzeugte Tachykardie vorliegt. In diesem Fall wird die Erzeugung eines Stimulationsimpulses aufgrund der nächsten, durch die Vorhof elektrode erfaßten P-Welle unterdrückt und damit der geschlossene Kreis aufgetrennt und die Tachykardie beendet.

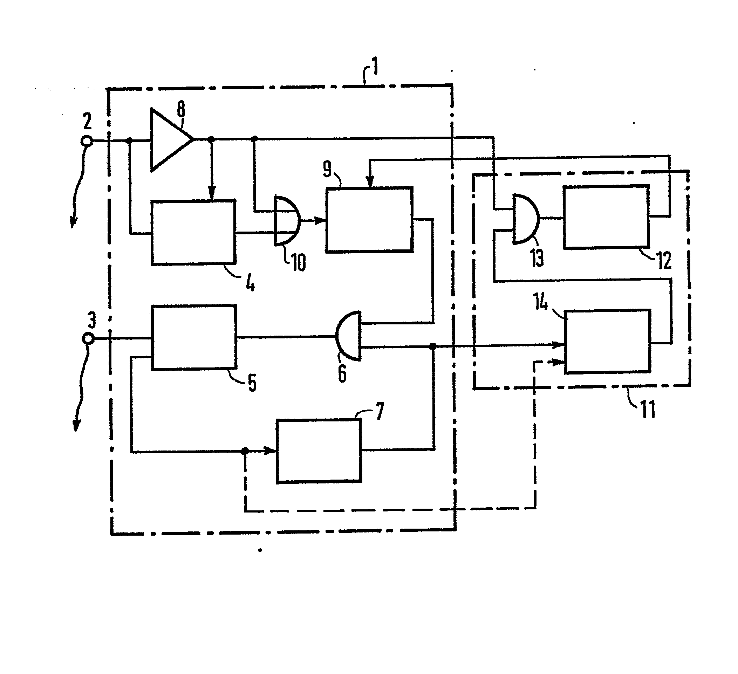

Die Erfindung ist nachfolgend anhand eines in der Zeichnung dargestellten Ausführungsbeispieles näher erläutert.

In der Zeichnung ist eine Stimulationsschaltung 1 dargestellt, die einen Eingang 2 für Atriumsignale und einen Ausgang 3 für Stimulationssignale aufweist. Die Erzeugung der Stimulationsimpulse erfolgt durch einen Impulsgenerator 4, der durch die P-Wellen am Eingang 2 synchronisiert wird und über eine Ausgangsstufe 5 die Stimulationsimpulse liefert. Der Ausgangsstufe 5 ist über ein UND-Gatter 6 eine Begrenzungsstufe 7 zugeordnet, die die höchste Synchronrate für den Herzschrittmacher festlegt, die beispielsweise bei 150 Pulsen pro Minute liegt. Die Stimulationsschaltung 1 enthält ferner noch einen Detektor 8 für die Rückstellung des Impulsgenerators 4 und eine Intervallschaltung 9, die über ein ODER-Gatter 10 mit dem Detektor 8 und dem Impulsgenerator 4 verbunden ist.

Der Stimulationsschaltung 1 ist eine Überwachungsschaltung 11 zugeordnet, die einen P-Wellenzähler 12 enthält, der über ein UND-Gatter 13 am Detektor 8 und an einem Fenstergenerator 14 angeschlossen ist. Der Fenstergenerator 14 ist mit seinem Eingang mit dem UND-Gatter 6 und gegebenenfalls mit der Begrenzungsstufe 7 verbunden.

Der Fenstergenerator 14 gibt dem P-Wellenzähler 12 ein Fenster vor, innerhalb dessen eine vorbestimmte Maximalzahl von n P-Wellen auftreten darf. Wird diese Zahl n erreicht, so wird die n + 1-te P-Welle über die Intervallschaltung 9 blockiert und der geschlossene Kreis aufge trennt, so daß die Tachykardie beendet wird.

Hinsichtlich der Wirkungsweise der in der Zeichnung dargestellten Stimulationsschaltung gilt im einzelnen folgendes:

Eine P-Welle, die vom Detektor 8 detektiert wird, stellt die Zeitbasiseinheit im Impulsgenerator 4 zurück, wenn sie außerhalb der atriellen Refraktärzeit liegt. Das Detektorsignal wird an die Intervallschaltung 9 weitergeleitet, wenn ein AV-Intervall gestartet wird. Am Ende des AV-Intervalles erreicht ein Signal die Ausgangsstufe 5, so daß dem Ventrikel ein Stimulationsimpuls zugeführt wird.

Die Begrenzungsstufe 7 dient zur Begrenzung der Frequenz des Herzschrittmachers auf eine höchste synchrone Rate (HSR). Ist die Begrenzungsstufe 7 aktiviert, wenn das der Ausgangsstufe 5 zugeführte Signal eintrifft, so läßt das UND-Gatter 6 dieses erst bei Freigabe durch die Begrenzungsstufe 7 durch. Die Begrenzungsstufe 7 erhält einen Puls von der Ausgangsstufe 5, wenn ein Stimulationsimpuls abgegeben wird, wobei ein HSR-Intervall gestartet wird. Die insoweit beschriebene Funktion ist bekannt.

Wesentlich für das dargestellte Ausführungsbeispiel der Erfindung ist nun, daß dann, wenn die Begrenzungsstufe 7 aktiviert wird, eine Zeitfunktion im Fenstergenerator 14 gestartet wird. Eine bestimmte Zeit nach dieser Aktivierung wird ein Fenster gebildet. Wird eine P-Welle während dieser Zeit detektiert, so zählt diese den P-Wellenzähler 12 um einen Schritt vorwärts. Wenn der P-Wellenzähler 12 seine n + 1-te Stufe erreicht hat, wird das AV-Intervall, das der n + 1-ten P-Welle zugeordnet wäre, gestoppt. Danach wird der P-Wellenzähler 12 auf Null zurückgesetzt.

Deshalb führt die n + 1-te P-Welle nicht zu einem Stimulationsimpuls und die gesamte Schaltung ist in ihren ursprünglichen Zustand zurückgeschaltet.

## Patentansprüche

1. Vorhofgesteuerter Herzschrittmacher mit einer Vorhofelektrode und einer Stimulationselektrode, **gekennzeichnet durch** eine Überwachungsschaltung (11) für die Frequenz der erzeugten P-Wellen, die die Weiterleitung einer P-Welle unterbricht, wenn diese Frequenz einen vorbestimmten Wert überschreitet.

2. Herzschrittmacher nach Anspruch 1, **dadurch gekennzeichnet,** daß die Überwachungsschaltung (11) einen P-Wellenzähler (12) und einen daran angeschlossenen Fenstergenerator (14) aufweist, der dem P-Wellenzähler ein Fenster vorgibt, innerhalb dessen eine vorbestimmte Höchstzahl n von P-Wellen auftreten darf und daß der P-Wellenzähler (12) und der Fenstergenerator (14) an der Stimulationsschaltung (1) derart angeschlossen sind, daß die Weiterleitung der n + 1-ten P-Welle beim Auftreten der n-ten P-Welle im Fenster blockiert wird.

## Claims

1. Auricle controlled heart pacemaker with an auricle electrode and a stimulating electrode, characterised by a monitoring circuit (11) for the frequency of the P-waves produced, which interrupts the passing on of a P-wave whenever this frequency exceeds a predetermined value.

2. Heart pacemaker according to Claim 1, characterised in that the monitoring circuit (11) has a P-wave counter (12) and a window generator (14) connected thereto, which gives the P-wave counter a virtual window, within which a predetermined maximum number n of P-waves may occur and in that the P-wave counter (12) and the window generator (14) are connected to the stimulating circuit (1) in such a way that the passing on of the n + 1-th P-wave is blocked when the n-th P-wave occurs in the window.

**Revendications**

1. Stimulateur cardiaque commandé par voie auriculaire, comportant une électrode auriculaire et une électrode de stimulation, caractérisé par un circuit (11) de contrôle de la fréquence des ondes P produites, qui interrompt la retransmission d'une onde P, lorsque cette fréquence dépasse une valeur prédéterminée.

2. Stimulateur cardiaque suivant la revendication 1, caractérisé par le fait que le circuit de contrôle (11) comporte un compteur d'ondes P (12) et un générateur de fenêtres (14), raccordé à ce compteur et prédéterminant, pour le compteur d'ondes P, une fenêtre, à l'intérieur de laquelle un nombre maximum prédéterminé n d'ondes P peut apparaître, et que le compteur d'ondes P (12) et le générateur de fenêtres (14) sont raccordés au circuit de stimulation (1) de telle sorte que la retransmission de la n + 1-ème onde P est bloquée lors de l'apparition de la n-ème onde P dans la fenêtre.